(19) Europäisches Patentamt
European Patent Office
Office européen des brevets

(11) Veröffentlichungsnummer: **0 003 230**
**B1**

# (12) EUROPÄISCHE PATENTSCHRIFT

(45) Veröffentlichungstag der Patentschrift: **08.04.81**

(51) Int. Cl.³: **C 07 C 47/52,** C 07 C 47/55, C 07 C 47/56, C 07 C 45/00, C 07 C 79/36, C 07 C 121/76

(21) Anmeldenummer: **78101723.1**

(22) Anmeldetag: **16.12.78**

## (54) Verfahren zur Herstellung von gegebenenfalls mehrfach substituierten o-Phthalaldehyden.

(30) Priorität: **30.12.77 CH 16298/77**
**04.04.78 CH 3601/78**

(43) Veröffentlichungstag der Anmeldung: **08.08.79 Patentblatt 79/16**

(45) Bekanntmachung des Hinweises auf die Patenterteilung: **08.04.81 Patentblatt 81/14**

(84) Benannte Vertragsstaaten: **BE CH DE FR GB IT NL SE**

(56) Entgegenhaltungen:
**ORGANIC SYNTHESIS, Vol. 34, 1945, J. Wiley & Sons, New York, Seiten 82–85**

(73) Patentinhaber: **CIBA-GEIGY AG, Patentabteilung Postfach, CH-4002 Basel (CH)**

(72) Erfinder: **Gosteli, Jacques, Dr., Anwilerstrasse 10, CH-4059 Basel (CH)**

**EP 0 003 230 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

BUNDESDRUCKEREI BERLIN

# Verfahren zur Herstellung von gegebenenfalls mehrfach substituierten o-Phthalaldehyden

Verfahren für die Herstellung von o-Phthalaldehyd sind in mehrfacher Weise in der Literatur schon seit langem beschrieben worden.

Zu erwähnen ist das in mehreren Stufen von J. Thiele und E. Winter, A 311, 360 (1900), verlaufende Oxydationsverfahren, welches von o-Xylol ausgehend über das Phthalaldehydtetraazetat verläuft. Die von F. Weygand und D. Tietjen in B 84, 625 (1951), beschriebene Methode liefert den o-Phthalaldehyd, wobei N,N,N',N'-Tetramethyl-o-phthalamid mittels Lithiumaluminiumhydrid als geeignetes Carbonsäurederivat reduziert wird. Eine Reduktion des verwendeten Tetramethyl-o-phthalamids kann nur unter peinlichem Feuchtigkeitsausschluß ausgeführt werden, so daß erhebliche verfahrenstechnische Schwierigkeiten in Kauf genommen werden müssen. Ein weiteres Verfahren gemäß F. Weygand, K. G. Kinkel und D. Tietjen in B 83, 394 (1950), geht vom o-Phthalalkohol aus. Die Oxydation des Alkohols erfolgt mit Selendioxid, wobei sich der Ester der selenigen Säure als Zwischenprodukt bildet, der erst durch vorsichtiges Erhitzen über offener Flamme zum Aldehyd umgewandelt wird. Bisher von Interesse war die durch J. Thiele und O. Günther, A 347, 106 (1906), beschriebene Hydrolyse des $\alpha,\alpha,\alpha',\alpha'$-Tetrabrom-o-xylols mittels Kaliumoxalat. Als nachteilig erwies sich die lange Reaktionszeit und die unbedingt notwendige Verwendung eines Lösungsgemisches aus Äthylalkohol und Wasser in relativ großen Volumenmengen. Auch die von F. Weygand et al., B 80, 391 (1947), beschriebene Hydrolyse des Tetrabrom-o-xylols kann als interessante Methode bezeichnet werden. Als nachteilig erweist sich jedoch hier die Verwendung von rauchender Schwefelsäure. Die durch Aufgießen auf Eis erhaltene volumenmäßig große Menge schwefelsaurer wäßriger Lösung muß mit großen Mengen Kochsalz gesättigt und mit großen Volumenmengen an Essigester ausgeschüttelt werden. Obgleich der o-Phthalaldehyd schon lange zu den bekannten Substanzen gehört, fehlt auch heute noch eine einfache, d. h. unter Verwendung von leicht zugänglichen Ausgangsstoffen und Reagentien verlaufende und wenig Stufen beanspruchende Synthese zur Herstellung dieser Verbindung, die diese in guter Ausbeute ergibt.

Die Erfindung betrifft ein besonders vorteilhaftes Verfahren zur Herstellung von gegebenenfalls substituierten o-Phthalaldehyden.

Das erfindungsgemäße Verfahren zur Herstellung eines gegebenenfalls substituierten o-Phthalaldehyds der Formel I

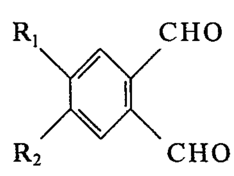

(I)

in der $R_1$ und $R_2$ unabhängig voneinander Wasserstoff, Niederalkyl, Hydroxy, Halogen, Nitro oder Carboxy bedeuten, besteht darin, daß man ein gegebenenfalls substituiertes $\alpha,\alpha,\alpha',\alpha'$-Tetrahalogen-o-xylol der Formel II,

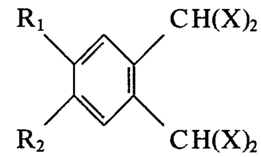

(II)

in der $R_1$ und $R_2$ die oben angegebene Bedeutung haben und X Chlor oder Brom bedeutet mit einem Carboxylat in wäßrigem Medium in Gegenwart eines Phasentransferkatalysators und einer anorganischen Base zum gegebenenfalls substituierten o-Phthalaldehyd hydrolysiert.

Falls $R_1$ und/oder $R_2$ Niederalkyl bedeuten, so können sie einen geraden oder verzweigten Niederalkylrest mit 1–7 Kohlenstoffatomen bedeuten und sind vorzugsweise Methyl, Äthyl, n-Propyl und Isopropyl, ferner n-Butyl, Isobutyl, sek. Butyl, tert. Butyl, n-Amyl, Isoamyl, n-Hexyl, Isohexyl oder n-Heptyl.

Falls $R_1$ und $R_2$ ein Halogenatom bedeuten, so kommen als Halogenatome solche bis zur Atomnummer 35 (Fluor, Chlor oder Brom) in Betracht.

Unter Carboxylat wird das Salz einer organischen Carbonsäure verstanden, wobei als Salze in erster Linie Metall-, wie Alkalimetall- oder Erdalkalimetallsalze, ferner Ammoniumsalze von solchen Säuren, insbesondere von entsprechenden aliphatischen, ferner auch aromatischen Carbonsäuren, wie von Niederalkancarbonsäuren, z. B. von Ameisen-, Essig- oder Propionsäure sowie von Benzoesäure in Frage kommen. Vorzugsweise verwendet man Alkalimetall-, wie Natrium- oder Kalium-, ferner auch Calciumniederalkanoate mit 1 bis 3 Kohlenstoffatomen unter Einbeziehung der Formiate, insbesondere die entsprechenden Formiate und Acetate und in erster Linie Natrium- oder Kaliumformiat oder -acetat.

Als Phasentransferkatalysator verwendet man vorwiegend Oniumsalze, insbesondere quaternäre Ammoniumsalze und Phosphoniumsalze, beispielsweise Tetraalkyl-ammoniumsalze bzw. -phosphoniumsalze, insbesondere entsprechende Halogenide, wie Tetra-n-butylammoniumchlorid oder -bromid bzw. Tetra-n-butylphosphoniumchlorid oder -bromid, Äthyltrioctylphosphoniumchlorid oder -bromid oder Arylniederalkyl-tri-niederalkylammoniumsalze, insbesondere entsprechende Halogenide, z. B. Benzyltriäthylammoniumchlorid. In analoger Weise können auch die entsprechenden Arsonium- und Sulfoniumsalze (E. V. Dehmlow, Ang. Chem., 89/8, 521–533) verwendet werden.

Zur Neutralisierung der dem Carboxylatreagens entsprechenden Säure, die bei der

Hydrolyse von Acylalzwischenprodukten entsteht, und zur Beschleunigung dieser Hydrolyse wird eine Base zugegeben. Man verwendet üblicherweise anorganische Basen, wie entsprechende metallische Basen, z. B. Alkalimetall- oder Erdalkalimetallbasen, insbesondere die entsprechende Hydroxide, Hydrogencarbonate oder vorzugsweise Carbonate, wie Natrium- oder Kaliumhydroxyd oder -hydrogencarbonat, und vorzugsweise Kalium- oder in erster Linie Natriumcarbonat. In analoger Weise können die entsprechenden Hydroxide, Hydrogencarbonat und Carbonate des Calciums oder Magnesiums verwendet werden, wobei Calciumcarbonat als schwerlösliche Base gegebenenfalls bevorzugt sein kann.

Die Reaktion wird in einem zweiphasigen System durchgeführt, das aus einer wäßrigen und einer organischen Phase besteht, wobei letztere aus dem $\alpha,\alpha,\alpha',\alpha'$-Tetrabrom- oder $\alpha,\alpha,\alpha',\alpha'$-Tetrachlor-o-xylol gebildet wird. Die erfindungsgemäß einzusetzenden Mengen der Carboxylat- und Phasentransferkatalysator-Verbindungen können erheblich variieren. Dem Reaktionsgemisch gibt man eine anorganische Base zur Neutralisierung der entstehenden Säure bei, die diese wieder in die Carboxylat-Verbindung überführt. Man könnte theoretisch katalytische Mengen, d. h. weniger als äquimolare Mengen, z. B. von etwa 1 Molprozent bis etwa 50 Molprozent (bezogen auf das Ausgangsmaterial) sowohl der Carboxylat-, als auch der Phasentransfer-Verbindung verwenden. Üblicherweise gibt man letztere in katalytischen Mengen, z. B. von etwa 1 Molprozent bis etwa 30 Molprozent (bezogen auf das Ausgangsmaterial) zu, wobei auch äquimolare Mengen oder ein Überschuß des Phasentransferkatalysators verwendet werden können. Die Carboxylatverbindung wird üblicherweise in etwa äquimolaren Mengen, gegebenenfalls in einem Unter-, und vorzugsweise in einem Überschuß angewandt. Vorzugsweise wird eine leicht-lösliche Base, wie ein Alkalimetallcarbonat, z. B. Natriumcarbonat, im Verlauf der Reaktion, üblicherweise in Portionen zugegeben. Falls notwendig, wird dabei das Reaktionsgemisch vor Zugabe der Base vorübergehend abgekühlt, z. B. um bei Verwendung eines Carbonats oder Hydrogencarbonats ein Überschäumen zu verhindern. Eine schwerlösliche Base kann jedoch auch von Anfang an im Reaktionsgemisch vorliegen, weshalb diese auch bevorzugt, beispielsweise Calciumcarbonat, verwendet wird.

Die Hydrolyse wird bei Verwendung einer leichtlöslichen Base, beispielsweise einer Alkalimetallbase, vorzugsweise in einem pH-Bereich von etwa 7,5 bis etwa 11 und in Gegenwart einer schwerlöslichen Base, beispielsweise eines Erdalkalicarbonates, wie z. B. Calciumcarbonat, in einem pH-Bereich unterhalb 7,5 ausgeführt; der pH-Wert sinkt je nach Löslichkeit des verwendeten Erdalkalicarbonates bis auf etwa 4.

Da das $\alpha,\alpha,\alpha',\alpha'$-Tetrabrom-o-xylol oder $\alpha,\alpha,\alpha',\alpha'$-Tetrachlor-o-xylol als Ausgangsmaterial in geschmolzenem Zustand die organische Phase des Zweiphasensystems bildet, wird die Reaktion vorzugsweise bei einer Temperatur oberhalb von 60°C, z. B. bei einer Temperatur von etwa 65°C bis etwa 160°C, vorzugsweise von 90°C bis etwa 140°C, ferner unter Normaldruck oder erhöhtem Druck, z. B. im Bombenrohr oder Autoklav, und/oder unter einer Inertgasatmosphäre, z. B. Stickstoff oder Argon, durchgeführt.

Die als Ausgangsmaterial verwendeten gegebenenfalls substituierten $\alpha,\alpha,\alpha',\alpha'$-Tetrabrom- oder $\alpha,\alpha,\alpha',\alpha'$-Tetrachlor-o-xylole sind bekannt und können z. B. durch Behandeln von o-Xylolen mit elementarem Brom unter Bestrahlen mit UV-Licht (Organic Syntheses, Coll. Vol. IV, p. 807 und 808) oder durch Behandeln von o-Xylolen mit elementarem Chlor unter Bestrahlung mit sichtbarem Licht (Houben—Weyl, Bd. 5/3, S. 739) hergestellt werden.

Im Vergleich zum nächstliegenden Stand der Technik, d. h. im Vergleich zu dem von F. Weygand et al., B 80, 391 (1947), beschriebenen Verfahren wird die Hydrolyse ohne rauchende Schwefelsäure ausgeführt. Die hierbei anfallende, mit Kochsalz gesättigte, große Menge wäßriger Lösung muß wiederum mit einer volumenmäßig großen Menge Essigester als Lösungsmittel ausgeschüttelt werden. Diese verfahrensmäßigen Nachteile liegen beim erfindungsgemäß ausführbaren Verfahren nicht vor.

Der erfindungsgemäß herstellbare o-Phthalaldehyd bzw. gegebenenfalls substituierte o-Phthalaldehyde werden beispielsweise als Reagens für die qualitative und quantitative Bestimmung von Ammoniak oder auch primären Aminen (Peptidchemie) verwendet. Sekundäre Amine reagieren mit den o-Phthalaldehyden nicht.

In den nachfolgenden Beispielen werden die Temperaturen in Celsiusgraden angegeben.

### Beispiel 1

Eine Suspension, bestehend aus 10,0 g $\alpha,\alpha,\alpha',\alpha'$-Tetrabrom-o-xylol (23,7 mMol), 9,7 g Natriumazetat (118 mMol), 1,74 g Tetrabutylammoniumbromid (5,4 mMol), 4,75 g Calciumcarbonat und 10 ml destilliertem Wasser wird in einer Stickstoffatmosphäre unter kräftigem Rühren zum Rückfluß erhitzt, wobei sich die Innentemperatur auf 118° einstellt. Nach 6stündiger Reaktionsdauer läßt man das Reaktionsgemisch abkühlen, fügt 50 ml Essigester hinzu und filtriert. Im Scheidetrichter wird die organische Phase abgetrennt und die wäßrige Phase nochmals mit Essigester gewaschen. Die vereinigten organischen Auszüge (Essigesterextrakte) werden mit einer Kochsalzlösung gewaschen, über Magnesiumsulfat getrocknet und anschließend im Vakuum eingeengt. Der erhaltene Rückstand wird in 40 ml Toluol aufgenommen und die Lösung an einer Säule, gefüllt mit Silicagel (100 g), chromatographisch gereinigt,

wobei nach dem Eindampfen des Eluates aus Toluol 2,10 g (66,2% d. Th.) o-Phthalaldehyd, Fp. 53–55°, isoliert werden.

Beispiel 2

Eine Suspension, bestehend aus 10,0 g $\alpha,\alpha,\alpha',\alpha'$-Tetrabrom-o-xylol, 8,0 g Natriumformiat, 1,74 g Tetrabutylammoniumbromid, 4,75 g Calciumcarbonat und 10 ml destilliertem Wasser wird in einer Stickstoffatmosphäre unter kräftigem Rühren 9 Stunden lang unter Rückfluß erhitzt. Nach dem Abkühlen werden 50 ml Essigester hinzugefügt und das Reaktionsgemisch wie unter Beispiel 1 weiterverarbeitet. Nach chromatographischer Reinigung (100 g Silicagel in der Säule) werden 2,54 g o-Phthalaldehyd (80,2% d. Th.), Fp. 53–55°, isoliert.

Beispiel 3

In Analogie zu Beispiel 1 wird eine Suspension, bestehend aus 5,78 g $\alpha,\alpha,\alpha',\alpha'$-Tetrachlorxylol (Reinheit ca. 95%), 9,62 g Natriumacetat, 1,74 g Tetrabutylammoniumbromid, 4,75 g Calciumcarbonat und 10 ml Wasser in einer Stickstoffatmosphäre unter Rühren 29 Stunden unter Rückfluß gekocht. Nach dem Abkühlen wird mit Wasser verdünnt, mit Essigester zweimal (je 100 ml) extrahiert, die vereinigten organischen Schichten mit gesättigter Kochsalzlösung gewaschen und über Magnesiumsulfat getrocknet. Das Lösungsmittel wird im Vakuum abdestilliert und der kristalline Rückstand in Toluol gelöst und an 100 g Silicagel chromatographiert. o-Phthaldialdehyd wird mit Toluol/Essigester = 19 : 1 eluiert, wobei nach dem Eindampfen des Eluates 2,30 g (72% d. Th.) gereinigter o-Phthalaldehyd erhalten werden.

**Patentansprüche**

1. Verfahren zur Herstellung eines gegebenenfalls substituierten o-Phthalaldehyds der Formel I,

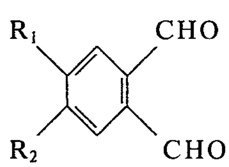

(I)

in der $R_1$ und $R_2$ unabhängig voneinander Wasserstoff, Niederalkyl, Hydroxy, Halogen, Nitro oder Carboxy bedeuten, dadurch gekennzeichnet, daß man ein gegebenenfalls substituiertes $\alpha,\alpha,\alpha',\alpha'$-Tetrahalogen-o-xylol der Formel II,

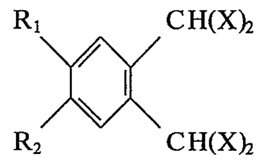

(II)

in der $R_1$ und $R_2$ die oben angegebene Bedeutung haben und X Chlor oder Brom bedeutet mit einem Carboxylat in wäßrigem Medium in Gegenwart eines Phasentransferkatalysators und einer anorganischen Base hydrolysiert.

2. Verfahren nach Patentanspruch 1, dadurch gekennzeichnet, daß man die Hydrolyse in Gegenwart eines quaternären Ammoniumsalzes oder Phosphoniumsalzes als Phasentransferkatalysator, einer metallischen Base und eines Alkali-, Erdalkali- oder Ammoniumsalzes einer organischen Säure ausführt.

3. Verfahren nach Patentanspruch 1 oder 2, dadurch gekennzeichnet, daß man die Hydrolyse in Gegenwart eines Tetraniederalkylammoniumsalzes, einer Alkalimetall- oder Erdalkalimetallbase und eines Alkali-, Erdalkali- oder Ammoniumsalzes einer aliphatischen Carbonsäure oder einer aromatischen Carbonsäure ausführt.

4. Verfahren nach einem der Patentansprüche 1 bis 3, dadurch gekennzeichnet, daß man die Hydrolyse in Gegenwart von Tetra-n-butylammoniumchlorid oder -bromid als Phasentransferkatalysator, eines Alkalimetall- oder Erdalkalimetallcarbonates und eines Alkalimetall- oder Erdalkalimetallformiates oder -acetates ausführt.

5. Verfahren nach Patentanspruch 1 oder 2, dadurch gekennzeichnet, daß man die Hydrolyse in Gegenwart von Benzyltriäthylammoniumchlorid oder -bromid, eines Alkalimetall- oder Erdalkalimetallcarbonates und eines Alkalimetall- oder Erdalkalimetallformiates oder -acetates ausführt.

6. Verfahren nach einem der Patentansprüche 1 bis 4, dadurch gekennzeichnet, daß man die Hydrolyse in Gegenwart von Tetra-n-butylammoniumchlorid oder -bromid, Calciumcarbonat und Natriumformiat oder -acetat ausführt.

7. Verfahren nach einem der Patentansprüche 1 bis 6, dadurch gekennzeichnet, daß man bei einer Temperatur über 60° arbeitet.

**Claims**

1. A process for the production of an unsubstituted or substituted o-phthalaldehyde of the formula I

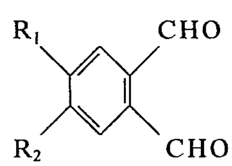

(I)

wherein $R_1$ and $R_2$, each independently of the

other, represent hydrogen, lower alkyl, hydroxyl, halogen, nitro, cyano or carboxyl, characterised in that an unsubstituted or substituted α,α,α',α'-tetrahalogeno-o-xylene of the formula II

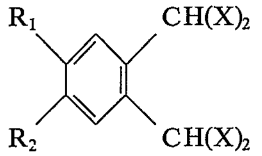

(II)

wherein $R_1$ and $R_2$ are as defined for formula (I) and X represents a halogen atom, is hydrolysed with a carboxylate in an aqueous medium in the presence of a phase transfer catalyst and an inorganic base.

2. A process according to claim 1, characterised in that the hydrolysis is carried out in the presence of a quaternary ammonium salt or phosphonium salt as phase transfer catalyst, and of a metal base, with an alkali metal salt, alkaline earth metal salt or ammonium salt of an organic acid.

3. A process according to either of claims 1 or 2, characterised in that the hydrolysis is carried out in the presence of a tetralower alkyl ammonium salt and an alkali metal base or alkaline earth metal base, and of an alkali metal salt, alkaline earth metal salt or ammonium salt of an aliphatic carboxylic acid or of an aromatic carboxylic acid.

4. A process according to any one of claims 1 to 3, characterised in that the hydrolysis is carried out in the presence of tetra-n-butylammonium chloride or tetra-n-butylammonium bromide as phase transfer catalyst, and of an alkali metal carbonate or alkaline earth metal carbonate, and of an alkali metal formate or acetate or an alkaline earth metal formate or acetate.

5. A process according to either of claims 1 or 2, characterised in that the hydrolysis is carried out in the presence of benzyltriethylammonium chloride or bromide and of an alkali metal carbonate or alkaline earth metal carbonate, and of an alkali metal formate or acetate or alkaline earth metal formate or acetate.

6. A process according to any one of claims 1 to 4, characterised in that the hydrolysis is carried out in the presence of tetra-n-butylammonium chloride or bromide and of calcium carbonate, with sodium formate or acetate.

7. A process according to any one of claims 1 to 6, characterised in that the process is carried out in the absence of an organic solvent at a temperature above 60° C.

**Revendications**

1. Procédé de préparation d'un aldéhyde o-phtalique éventuellement substitué de formule I

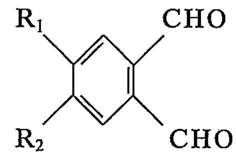

(I)

dans laquelle $R_1$ et $R_2$ représentent chacun, indépendamment l'un de l'autre, l'hydrogène, un groupe alkyle inférieur, hydroxy, un halogène, un groupe nitro ou carboxy, caractérisé en ce que l'on hydrolyse un α,α,α',α'-tétrahalogéno-o-xylène éventuellement substitué de formule II

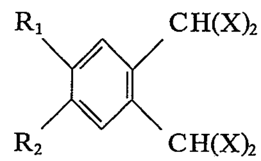

(II)

dans laquelle $R_1$ et $R_2$ ont les significations indiquées ci-dessus et X représente le chlore ou le brome, à l'aide d'un carboxylate en milieu aqueux en présence d'un catalyseur de transfert de phase et d'une base anorganique.

2. Procédé selon la revendication 1, caractérisé en ce que l'on effectuel'hydrolyse en présence d'un sel d'ammonium ou d'un sel de phosphonium quaternaire servant de catalyseur de transfert de phase, d'une base métallique et d'un sel alcalin, alcalino-terreux ou d'ammonium d'un acide organique.

3. Procédé selon la revendication 1 ou 2, caractérisé en ce que l'on effectue l'hydrolyse en présence d'un sel de tétra-(alkyl inférieur)-ammonium, d'une base de métal alcalin ou alcalino-terreux et d'un sel alcalin, alcalino-terreux ou d'ammonium d'un acide carboxylique aliphatique ou d'un acide carboxylique aromatique.

4. Procédé selon l'une des revendications 1 à 3, caractérisé en ce que l'on effectue l'hydrolyse en présence de chlorure ou bromure de tétra-n-butylammonium servant de catalyseur de transfert de phase, d'un carbonate de métal alcalin ou alcalino-terreux et d'un formiate ou acétate de métal alcalin ou alcalino-terreux.

5. Procédé selon la revendication 1 ou 2, caractérisé en ce que l'on effectue l'hydrolyse en présence de chlorure ou bromure de benzyl-triéthyl-ammonium, d'un carbonate de métal alcalin ou alcalino-terreux et d'un formiate ou acétate de métal alcalin ou alcalino-terreux.

6. Procédé selon l'une des revendications 1 à 4, caractérisé en ce que l'on effectue l'hydrolyse en présence de chlorure ou bromure de tétra-n-butylammonium, de carbonate de calcium et de formiate ou acétate de sodium.

7. Procédé selon l'une des revendications 1 à 6, caractérisé en ce que l'on opère à une température de 60°.